# EUROPÄISCHE PATENTSCHRIFT

(11) **EP 2 734 488 B1**
(45) Veröffentlichungstag und Bekanntmachung des Hinweises auf die Patenterteilung: **05.10.2016**
(21) Anmeldenummer: 12732533.0
(22) Anmeldetag: 22.06.2012
(51) Int. Cl.: C07C 29/74, C07C 31/20, C07C 29/149, C07B 57/00, C12P 41/00, C12P 7/18

(54) **HERSTELLUNG VON OPTISCH REINEM PROPAN-1,2-DIOL**
PRODUCTION OF OPTICALLY PURE PROPANE-1,2-DIOL
PRÉPARATION DE PROPANE-1,2-DIOL OPTIQUEMENT PUR

(30) Priorität: 20.07.2011 DE 102011107959
(43) Veröffentlichungstag der Anmeldung: 28.05.2014
(73) Patentinhaber: ThyssenKrupp Industrial Solutions AG, 45143 Essen (DE)
(72) Erfinder: TIETZ, Wolfgang, 06408 Biendorf (DE); SCHULZE, Joachim, 59494 Soest (DE); BÖRNER, Armin, 18059 Rostock (DE); SHUKLOV, Ivan, 18059 Rostock (DE); KÜHLEIN, Klaus, 65779 Kelkheim (DE)
(74) Vertreter: ThyssenKrupp Intellectual Property GmbH
(86) Internationale Anmeldenummer: PCT/EP2012/002638
(87) Internationale Veröffentlichungsnummer: WO 2013/010618

(56) Entgegenhaltungen:
- WO-A2-03/083126
- WO-A2-2006/124899
- BOGÃ R KRISZTIÃ N ET AL: "Large-scale ruthenium- and enzyme-catalyzed dynamic kinetic resolution of (rac)-1-phenylethanol", BEILSTEIN JOURNAL OF ORGANIC CHEMISTRY, BIOMED CENTRAL, LONDON, GB, Bd. 3, Nr. 1, 20. Dezember 2007 (2007-12-20), Seite 50, XP021041124, ISSN: 1860-5397

## Beschreibung

Die Erfindung betrifft ein Verfahren zur Herstellung von optisch reinem Propan-1,2-diol ausgehend von Lactiden.

Propan-1,2-diol wird industriell durch Hydrolyse von Propylenoxid oder auch ausgehend von Glycerin hergestellt. Es wird hauptsächlich in Kosmetikartikeln, wie Hautcremes und Zahnpasta eingesetzt. Es verbessert die Resorption verschiedener Wirkstoffe und weist eine antimikrobielle Wirksamkeit auf. Außerdem ist es in der EU als Lebensmittelzusatzstoff zugelassen. Es dient auch als Trägerstoff und Trägerlösungsmittel für Farbstoffe, Antioxidationsmittel und Emulgatoren.

Bei Lactiden handelt es sich um zyklische Diester der Milchsäure. Beispielsweise bei der Milchsäurepolymerisation können verschiedene Formen von Lactiden anfallen. Dies können reines L,L-Lactid oder reines D,D-Lactid sein. Wegen der vorherrschenden hohen Temperaturen, die für einen schnellen Reaktionsablauf benötigt werden, sowie aufgrund von kationischen Verunreinigungen der Milchsäure oder der Reaktionsgefäße (z.B. durch Korrosion) besteht das Problem der Racemisierung, wodurch meso-Lactid als Nebenprodukt entsteht. Meso-Lactid ist wie L,L-Lactid ein zyklischer Diester mit zwei optisch aktiven Kohlenstoffatomen im Ring. Es besitzt ein optisches R- und ein S-Zentrum und ist daher optisch inaktiv. Meso-Lactide beeinflussen eine sich anschließende Milchsäurepolymerisation negativ und müssen somit abgetrennt werden und fallen damit als Nebenprodukt bei der Milchsäurepolymerisation an.

Des Weiteren gibt es rac-Lactide, die beispielsweise aus gleichen Mengen D,D-Lactid und L,L-Lactid durch Verschmelzen gewonnen werden. Die einzelnen Lactide unterscheiden sich durch ihre Schmelztemperaturen. So weist das L,L-Lactid und das D-D-Lactid Schmelztemperaturen von 97°C auf, während das meso-Lacitd eine Schmelztemperatur von 54°C und das L,L/D,D-Lactid eine Schmelztemperatur von 129°C aufweisen.

Die Hydrierung von Alkylestern der Milchsäure zu Propan-1,2-diol ist bekannt. Diese Transformation ist mit heterogenen als auch homogenen Katalysatoren möglich.

Die Hydrierung von Milchsäureethylester wurde z.B. in Ethanol mit einem Kupferoxid-Chromoxid-Katalysator bei 125 °C und einem H₂-Druck von 345 bar beschrieben (H. Adkins et al, J. Am. Chem. Soc. 1948, 70, 3121 - 3125). Die Verwendung eines Kupferoxid-Chromoxid-Barium-Katalysators bei 250 °C und 300 bar Wasserstoffdruck war ebenfalls erfolgreich (K. Folkers et al, J. Am. Chem. Soc. 1932, 54, 1145 - 1154). Erst kürzlich wurde in WO 2011036189 A1 und WO 2009103682 A1 die Hydrierung von Milchsäureestern in der Gasphase mit Kupfersilikaten beschrieben. Kupfer auf Aluminiumoxid wurde in WO 2005023737 A1 ebenfalls für die Reduktion von Milchsäuremethylester vorgeschlagen.

Darüber hinaus wurde eine Reihe von heterogenen Rutheniumkatalysatoren untersucht. Zum Beispiel ist Ru-B geträgert auf Titanoxid ein aktiver Katalysator für die Hydrierung von Milchsäureethylester in Wasser als Lösungsmittel bei 90 °C und 40 bar H₂ (G.-Y. Fan et al, Chem. Lett. 2008, 37, 852 - 853). Der Katalysator wurde durch die Reduktion von RuCl₃ mit NaBH₄ präpariert. RuB auf Zinn-modifizierten SBA-15 Molekularsieb (G. Luo et al, Appl. Catal., A: General 2007, 332, 79-88) und Ru-B auf γ-Aluminiumoxid (G. Luo et al, J. Mol. Catal. A: Chemical 2005, 230, 69-77 und G. Luo et al, Appi. Catal., A: General 2004, 275, 95 - 102) führten ebenfalls zu durchschnittlichen bis guten Umsätzen in der Reduktion von Milchsäureethylester. Leider sind die Ru-B Katalysatoren nicht chemoselektiv. Ein Nishimura-Katalysator (Rh/Pt-Oxid) erwies sich als effizient in der Hydrierung von Milchsäuremethylester bei 25 °C und 100 bar Wasserstoffdruck in MeOH (M. Studer et al, Adv. Synth. Catal. 2001, 343, 802 - 808). Auch homogene RutheniumKatalysatoren mit modifizierenden P,N-Liganden (EP2161251 A1; W. Kuriyama et al, Adv. Synth. Catal. 2010, 352, 92 - 96) oder P,P-Liganden (EP 1970360 A1) wurden sehr erfolgreich für die Hydrierung von Milchsäureestern eingesetzt, wobei die Reaktionen bei Temperaturen von 80-90 °C und H₂-Drücken von 30-50 bar H₂ abliefen.

Erst vor kurzem gelang die Reduktion von Lactid zum Propandiol-d₂ mit Hilfe von Lithiumaluminiumdeuterid im Rahmen mechanistischer Studien (R. M. Painter et al, Angew. Chem. Int. Ed. 2010, 49, 9456-9459). WO0308312 offenbart ein Verfahren zu Herstellung von Propan-1,2-diol durch Hydrolyse von Propylenoxid und enzymatische Racematspaltung.

WO2006/124899 beschreibt die katalytische Hydrierung von Lactiden zu Propylenglykol. Dabei wird die Hydrierung entweder in der Gasphase oder in der Flüssigphase, in Gegenwart von z.B. aliphatischen Alkoholen, durchgeführt. Dabei werden Reaktionsbedingen von 20°C bis 250°C und 1,4 bis 275 bar zugrunde gelegt und die Reaktionszeit beträgt 1 bis 10 Stunden. Bei dieser Reaktion spielt es keine Rolle, ob das Ausgangsprodukt eines der Enantiomere oder ein Gemisch davon ist. Es ist aber davon auszugehen, dass es bei der Reaktion zu einer Racemisierung kommt und das erhaltene Propylenglykol deshalb nicht in optisch reiner Form erhalten wird.

Dies ist für viele Anwendungen nachteilig, da beide Enantiomere zwar gleiche physikalische Eigenschaften besitzen, aber in chemischen Reaktionen, bei denen ein anderer enantiomerenreiner Reaktionspartner beteiligt ist, unterschiedlich reagieren. Auch beim Einsatz im pharmakologischen Bereich und für Anwendungen im Bereich der Agrochemie, der Düfte und der Geschmäcker rufen zueinander enantiomere Stoffe unterschiedliche Wirkungen hervor.

Um aus racemischen Mischungen ein Enantiomer in seiner optisch reinen Form zu erhalten ist die dynamisch kinetische Racematspaltung (DKR) bekannt. Zur Racematspaltung von Alkoholen werden nur sehr geringe Mengen eines Ru-Katalysators (bis zu 0,05 mol%) benötigt (K. Bogar et al, Beilstein J. Org. Chem 2007, 3 (50)). Dabei handelt es sich um eine kinetische Racematspaltung mit einer in situ Racemisierung des Substrates. Die Racematspaltung erfolgt enzymatisch durch Biokatalyse und die Racemisierung wird durch Metallkatalysatoren, aber auch durch Organokatalysatoren, Basen, Erhitzen, dem Einsatz von Enzymen, Lewis-Säuren sowie Redox- und Radikalreaktionen erreicht. Die Anwendung des Verfahrens zur Herstellung von Propan-1,2-diol in optisch reiner Form ausgehend von Lactiden ist allerdings nicht bekannt.

Aus diesem Grund wäre es wünschenswert ein Verfahren bereitzustellen, das es erlaubt Propan-1,2-diol in optisch reiner Form zu generieren. Dieses Verfahren sollte außerdem von Lactiden ausgehen, da gerade meso-Lactid als Abfallprodukt bei der Milchsäurepolymerisation anfällt und somit weiterverwendet werden könnte. Aber auch die anderen oben erwähnten Lactidformen könnten damit vorteilhaft zu optisch reinem Propan-1,2-diol umgewandelt werden.

Aufgabe der Erfindung ist es deshalb ein Verfahren bereitzustellen, das die Herstellung von optisch reinem Propan-1,2-diol in einem Bereich von von ≥ 99 % e.e. ausgehend von Lactiden, erlaubt.

Die Erfindung wird gelöst durch ein Verfahren zur Herstellung von optisch reinem Propan-1,2-diol umfassend die Verfahrensschritte
a. Hydrierung von Lactiden, wobei eine metallkatalysierte heterogene Katalyse in Gegenwart von Wasserstoff durchgeführt wird, wobei ein Rohprodukt, enthaltend Propan-1,2-diol, hergestellt wird, und
b. dynamisch kinetische Racematspaltung, wobei in einem Bereich von ≥ 99 % e.e. optisch reines Propan-1,2-diol hergestellt wird.

Dabei läuft in Schritt a) die nachfolgende Reaktion ab:

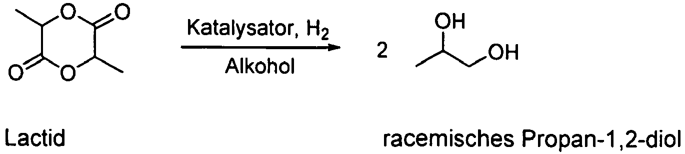

Der Alkohol fungiert dabei sowohl als Lösungsmittel als auch als Reaktant, wobei die Konzentration von Lactid in Alkohol unkritisch für die erzielte Ausbeute ist. Bevorzugt sollte der Alkohol im Überschuss vorhanden sein.

Das zur dynamisch kinetischen Racematspaltung angewandte System besteht aus einem Katalysator, der das vorgelagerte racemisierungsgleichgewicht einstellt und einem Enzym, das eines der gebildeten Enantiomere durch Veresterung dem Racemisierungsgleichgewicht entzieht.

Mit dem Begriff "optisch rein" ist im Sinne dieser Anmeldung enantiomerenreines Propan-1,2-diol gemeint. D.h. die Herstellung von > 99 % e.e. optisch reinem Propan-1,2-diol, wie im Hauptanspruch vorgesehen, kann mit einer > 99 %iger Enantiomerenreinheit gleichgesetzt werden. Es spielt dabei keine Rolle ob das (R)-Enantiomer oder das (S)-Enantiomer hergestellt werden.

In einer Ausgestaltung des erfindungsgemäßen Verfahrens werden die Lactide ausgewählt aus der Gruppe umfassend D,D-Lactid, L, L-Lactid, meso-Lactid und L,L/D,D-Lactid eingesetzt. Bei Lactiden handelt es sich um zyklische Ester von Milchsäuren, die in Form von Enantiomeren, also in D- bzw. L-Form auftreten können, L,L-Lactid bezeichnet einen Ester bestehend aus zwei L-Milchsäuren und wird in der Literatur auch als S,S-Lactid bezeichnet. Analoges gilt für das D,D-Lactid, das auch als R,R.Lactid bezeichnet wird. Unter L,L/D,D-Lactid wird das Racemat (in der Literatur auch als rac-Lactid oder R,S-Lactid bezeichnet) bestehend aus der äquimolaren Mischung aus D,D- und L,L-Lactid verstanden. Im Unterschied bezeichnet meso-Lactid, ein Lactid bestehend aus D- und L-Milchsäure. In Anspruch 2 wird somit dargelegt, dass alle möglichen Lactide dem erfindungsgemäßen Verfahren unterworfen werden können, darunter auch Oligolactide unterschiedlicher Milchsäureenantiomerenzusammensetzung und bevorzugt Dilactide.

Mit Vorteil wird die metallkatalysierte heterogene Katalyse in Schritt a) in der Flüssigphase durchgeführt. Bevorzugt wird die Flüssigphase dabei ausgewählt aus einer Gruppe Lösungsmittel umfassend, Wasser, aliphatische oder aromatische Kohlenwasserstoffe aufweisend eine Kettenlänge von bis zu 10 C-Atomen und Mischungen davon, wobei die aliphatischen Kohlenwasserstoffe bevorzugt Alkohole sind, und besonders bevorzugt Methanol und/oder Ethanol eingesetzt werden.

In bevorzugter Ausführungsform des erfindungsgemäßen Verfahrens wird die heterogene Katalyse in Schritt a) mittels eines Katalysators aus der Gruppe der Metalle durchgeführt, wobei das Metall aus einer Gruppe umfassend Ruthenium, Rhodium, Rhenium, Palladium, Platin, Nickel, Kobalt, Molybdän, Wolfram, Titan, Zirkonium, Niobium, Vanadium, Chrom, Mangan, Osmium, Iridium, Eisen, Kupfer Zink, Silber, Gold, Barium und Mischungen davon ausgewählt wird, und bevorzugt Kupferchromit-Katalysatoren und/oder mit Barium versetzte Kupferchromit-Katalysatoren eingesetzt werden.

In weiterer Verfahrensausgestaltung wird die heterogene Katalyse in Schritt a) unter 20 bis 300 bar Wasserstoffdruck, bevorzugt unter 130 bis 170 bar Wasserstoffdruck und besonders bevorzugt unter 140 bis 160 bar Wasserstoffdruck durchgeführt.

Vorzugsweise wird die heterogene Katalyse in Schritt a) in einem Temperaturbereich von 20°C bis 250°C, bevorzugt in einem Temperaturbereich von 130°C bis 170°C, und besonders bevorzugt in einem Temperaturbereich von 145°C bis 155°C durchgeführt.

Optional wird vor der Durchführung der heterogenen Katalyse in Schritt a) der Druckbehälter 1 bis 5 mal und bevorzugt 3 mal mit Wasserstoff gespült.

In weiterer Ausgestaltung des Verfahrens wird die heterogene Katalyse in Schritt a) über einen Zeitraum von 5 bis 20 Stunden, bevorzugt über einen Zeitraum von 10 bis 18 Stunden, und besonders bevorzugt über einen Zeitraum von 12 bis 16 Stunden durchgeführt.

Mit Vorteil wird bei der heterogenen Katalyse in Schritt a) gerührt. Außerdem ist es weiterhin vorteilhaft, wenn bei der heterogenen Katalyse in Schritt a) kontinuierlich Wasserstoff nachgepresst wird.

In bevorzugter Ausführungsform des Verfahrens wird der Katalysator nach Beenden der heterogenen Katalyse in Schritt a) vom Rohprodukt getrennt.

In weiterer Ausgestaltung wird das Rohprodukt, das aus Schritt a) resultiert, einem Aufkonzentrierungsschritt und/oder einem Destillationsschritt unterzogen, wobei eine Fraktion enthaltend Propan-1,2-diol und eine Fraktion enthaltend Lösungsmittel generiert werden.

Bevorzugt wird das Lösungsmittel, das zur heterogenen Katalyse in Schritt a) eingesetzt wird, in das Verfahren zurückgeführt.

Eine weitere Ausführungsvariante des Verfahrens ist es, das Propan-1,2-diol, das aus Schritt a) erhalten wird, mit einer Schutzgruppe zu versehen und 1-0-substituiertes Propandiol zu erzeugen. Mit Vorteil ist die Schutzgruppe eine rezyklisierbare, achirale Schutzgruppe und wird ausgewählt aus der Gruppe umfassend *tert*-Butyl, Phenyl, Methyl, Acetyl, Benzoyl, Trityl, Silyl und Benzyl. Somit können also Pivalate, p-Methoxybenzyl, Trimethylsilyl, Triethylsilyl, Triisopropylsilyl, Diphenylmethylsilyl oder Di-tert-Butylmethylsilyl verwendet werden. Im Prinzip kann jede achirale Schutzgruppe genutzt werden (T.W. Green et al, Protective Groups in Organic Synthesis, Wiley-Interscience, New York, 1999). Besonders bevorzugt ist die Schutzgruppe *tert*-Butyl an der primären Hydroxylgruppe des Propan-1,2-diols aus Schritt a).

In weiterer Ausgestaltung wird in Schritt b) zur dynamisch kinetischen Racematspaltung eine enzymatische Racematspaltung in Gegenwart eines Metallkatalysators durchgeführt. Dabei werden bevorzugt Lipasen eingesetzt. Als Metallkatalysatoren kommen vorzugsweise Rutheniumkatalysatoren in Betracht. Besonders bevorzugt werden Rutheniumkatalysatoren mit immobilisierten Lipasen eingesetzt.

Bevorzugt wird die dynamisch kinetische Racematspaltung in Schritt b) in einem Temperaturbereich von 60°C bis 90°C durchgeführt. Dabei beträgt die Reaktionszeit 30 bis 200 h, bevorzugt 40 bis 60 h.

In weiterer Ausgestaltung wird die dynamisch kinetische Racematspaltung in Schritt b) in Gegenwart von Na₂CO₃ durchgeführt, wobei das Na₂CO₃ in einer Menge von 0,4 mmol bis 5 mmol pro 33 mg Enzym zugesetzt wird, dies entspricht 330 units. Dabei ist Na₂CO₃ praktisch unlöslich im Reaktionsmedium und wirkt als heterogenes Additiv. Das Enzym ist dabei mit Vorteil Novozym 435.

Nachfolgend soll die vorliegende Erfindung anhand einiger Ausführungsbeispiele detailliert erläutert werden.

### Beispiel 1: Hydrierung des rac-Lactids mit einem Cu/Cr Katalysator

L,L/D,D-Lactid (1.00 g, 6.9 mmol) und Kupferchromit (1.33 g, 133 wt%) werden in einem 10 mL Autoklav in 5 ml abs. MeOH suspendiert. Der Autoklav wird dreimal mit H₂ gespült. Anschließend werden 150 bar Wasserstoff aufgepresst. Die Reaktionsmischung wird bei 150 °C 15 Stunden lang gerührt. Der Wasserstoff wird kontinuierlich nachgepresst, wobei ein Druck zwischen 148 und 153 bar aufrecht erhalten wird. Nach dem Abkühlen und Belüften des Autoklaven wird die Reaktionsmischung mit 5 mL MeOH verdünnt und vom Katalysator abzentifugiert (75 min, 4500 rpm). Die blau-grüne Reaktionslösung wird dekantiert, der Rückstand mit 3 mL MeOH nachgewaschen und im Vakuum bei 40 °C und 40 mbar aufkonzentriert. Das Rohprodukt (2.06 g) hat eine dunkelblaue Farbe und besteht aus Propan-1,2-diol verunreinigt mit ca. 5 % MeOH (¹³C-NMR Spektrum). Das reine Produkt (0.68 g, 68 %) wird als farblose Flüssigkeit nach der Destillation bei 101-102 °C und 8 mbar erhalten. Der anorganische Rückstand nach der Destillation beträgt ca. 30 mg.

### Beispiel 2: Hydrierung des rac-Lactids mit einem Cu/Cr/Ba-Katalysator

L,L/D,D-Lactid (1.00 g, 6.9 mmol) und mit Barium dotiertes Kupferchromit (1.33 g, 133 wt%) werden in einem 10 mL Autoklav in 5 ml abs. MeOH oder EtOH suspendiert. Der Autoklav wird dreimal mit H₂ gespült. Anschließend werden 150 bar Wasserstoff aufgepresst. Die Reaktionsmischung wird bei 150 °C 12 Stunden lang gerührt. Der Wasserstoff wird kontinuierlich nachgepresst, wobei ein Druck zwischen 148 und 153 bar aufrecht erhalten wird. Nach dem Abkühlen und Belüften des Autoklaven wird die Reaktionsmischung mit 5 mL MeOH verdünnt und der Katalysator abzentrifugiert (15 min, 4500 rpm). Die Reaktionslösung wird im Vakuum bei 40 °C und 40 mbar aufkonzentriert. Das Rohprodukt (1.03 g) hat eine leichte blaue Farbe und besteht aus Propan-1,2-diol verunreinigt noch mit ca. 5 % MeOH. Dies wurde über ein ¹³C-NMR Spektrum ermittelt (nicht gezeigt). Das reine Produkt (0,8 g, 82 %) wird als farblose Flüssigkeit durch Destillation bei 101-102 °C und 8 mbar gewonnen. Die Reaktion mit EtOH verläuft dabei deutlich langsamer als in MeOH.

Der Cu/Cr/Ba-Katalysator weist den Vorteil auf, dass die Reaktion im Vergleich zum Cu/Cr-Katalysator beschleunigt abläuft. Dies wurde aus Wasserstoffverbrauchskurven, die während der Versuche aufgenommen wurden ermittelt. Daraus folgte, dass die die Hydrierung mit dem Cu/Cr/Ba-Katalysator um ca. 20% schneller abläuft. Außerdem löst sich praktisch kein Katalysator im Fall des Cu/Cr/Ba-Katalysators in der Reaktionslösung, was bedeutet, dass die Reaktion völlig heterogen abläuft. Im Unterschied dazu waren bis zu 30 mg aus einer Gesamtmenge von 1,3 g Cu/Cr-Katalysator nach einem Hydrierungsversuch in der Reaktionslösung enthalten.

### Beispiel 3: Hydrierung weiterer Lactidformen mit einem Cu/Cr/Ba-Katalysator.

Die Durchführungsweise entsprach wie in Beispiel 2 beschrieben in Gegenwart von 5 ml MeOH unter 150 bar H₂ und unter Verwendung des Cu/Cr/Ba-Katalysators. Die genauen Reaktionsbedingungen können Tabelle 1 entnommen werden.

**Tabelle 1:**

| Lauf | Substrat | Ansatz [g] | Zeit [h] | Temperatur [°C] | GC-Ausbeute [%] |
|---|---|---|---|---|---|
| 1 | rac-Lactid | 1,0 | 15 | 150 | 100 |
| 2 | L, L-Lactid | 1,0 | 15 | 150 | 100 |
| 3 | meso-Lactid | 1,0 | 15 | 150 | 100 |

| | | | | | |
|---|---|---|---|---|---|
| Aus Tabelle 1 geht hervor, dass sämtliche Formen von Lactiden, also auch das meso-Lactid, das als Abfallprodukt bei der Milchsäurepolymerisation anfällt, zu 100 % umgesetzt werden kann. Somit ist das erfindungsgemäße Verfahren geeignet meso-Lactide zu Propan-1,2-diol zu verarbeiten. Meso-Lactiden, das noch mit Resten von Milchsäure verunreinigt war, konnte nicht zu Propan-1,2-diol umgesetzt werden. Aus diesem Grund ist es erforderlich die für die Hydrierung eingesetzten Lactide in reiner bzw. in aufgereinigter Form zu verwenden. | | | | | |

### Beispiel 4: Untersuchung des Racemisierungsgrades des mittels Hydrierung hergestellten Propan1,2-diols

Zur Derivatisierung des aus den Hydrierungen erzeugten Propan-1,2-diols wurden 0,28 g (3,7 mmol) Propan-1,2-diol zu 1,2 ml Phenylisocyanat (11 mmol) gegeben. Die Reaktionsmischung wurde 30 min bei 100°C erhitzt und dann auf Raumtemperatur abgekühlt. Anschließend wurde Diethylether (5 ml) zugesetzt. Die so entstandenen weißen Kristalle wurden abfiltriert und mit 50 ml Hexan nachgewaschen. Das daraus resultierende Produkt wurde für die Enantiomerenanalytik verwendet, wozu es an einer chiralen HPLC-Säule CHIRALCEL®OD-H in Heptan/EtOH 80 : 20 aufgetrennt wurde.

Die Ergebnisse bei Einsatz von L,L-Lactid, das nach der Vorschrift des Beispiels 2 generiert wurde, sind in Tabelle 2 dargestellt.

**Tabelle 2:**

| Lauf | Substrat | Ansatz [g] | Zeit [h] | Temperatur [°C] | Umsatz [%] | e.e. [%] |
|---|---|---|---|---|---|---|
| 1 | L,L-Lactid | 1,0 | 12 | 125 | 90 | 88 |
| 2 | L,L-Lactid | 0,5 | 12 | 150 | 100 | 0 |

| | | | | | | |
|---|---|---|---|---|---|---|
| Aus Tabelle 2 geht hervor, dass die bei der Hydrierung erhaltene Enantiomerenreinheit des erzeugten Propandiols von der Temperatur abhängig ist. Bei einer Temperatur von 150°C erhält man ausschließlich ein racemisches Gemisch. Bei 125°C beträgt der e.e.-Wert 88 %. Somit entsteht bei der Hydrierung der Lactide also ein racemisches Gemisch an Propan-1,2-diol. Bei weiterer Absenkung der Temperatur besteht die Gefahr, dass die Hydrierungsreaktion zum Erliegen kommt. | | | | | | |

### Beispiel 5: Dynamisch kinetische Racematspaltung zur Herstellung von optisch reinem Propan-1,2-diol

Beispielhaft wurde t*ert*-Butyl als Schutzgruppe eingeführt und *tert*-Butyloxypropan-2-ol aus dem durch die Hydrierung gewonnen racemischen Gemisch von Propan-1,2-diol gewonnen. Die enzymatische Racematspaltung läuft dann nach folgendem Schema ab:

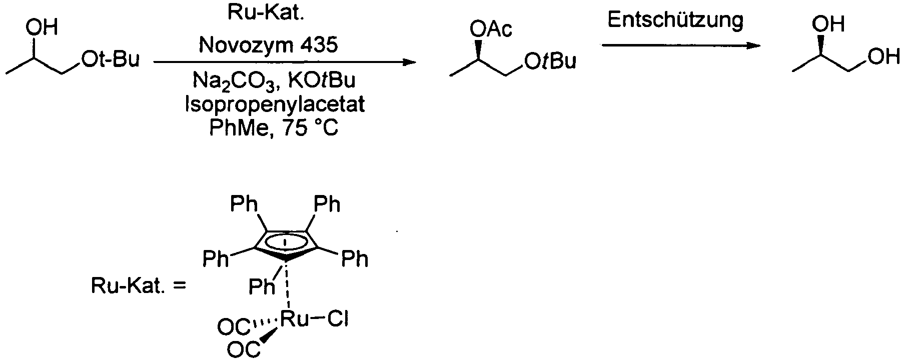

Die Reaktion wurde in 7,5 ml Toluol bei 75°C durchgeführt. Es wurden dazu 20 mmol Isopropenylacetat, 19,8 mmol 1-*tert*-Butoxypropanol-2, 0,02 mmol (Ph₅Cp)Ru(CO)₂Cl, 0,04 mmol t-BuOK, 50 mg Na₂CO₃vermischt. Die Ergebnisse sind in Tabelle 3 dargestellt:

**Tabelle 3:**

| Lauf | Zeit [h] | Novozym 435 [mg] | Umsatz [%] | e.e. [%] |
|---|---|---|---|---|
| 1 | 68 | 13 | 60 | 99 |
| 2 | 42 | 33 | 66 | 99 |
| 3 | 90 | 33 | 80 | 99 |
| 4 | 190 | 33 | 85 | 99 |

| | | | | |
|---|---|---|---|---|
| Aus Tabelle 3 geht hervor, dass schon 13 mg Novozym 435 (Lauf 1) ausreichend sind, um eine exzellente Stereoselektivität von > 99 % e.e. zu erhalten. Allerdings sollte der Umsatz weiter gesteigert werden und es wurde mit der 2,5fachen Menge an Enzym gearbeitet (Lauf 2 - 4). Es wurde beobachtet, dass sich die rutheniumkatalysierte Epimerisierung mit einer größeren Menge an Enzym zwar verlangsamt, der Umsatz steigt aber an. | | | | |

### Beispiel 6: Dynamisch kinetische Racematspaltung zur Herstellung von optisch reinem Propan-1,2-diol bei weiter verbessertem Umsatz

Die Reaktion wurde in 20 ml Toluol bei 75°C durchgeführt. Es wurden dazu 20 mmol Isopropenylacetat, 19,8 mmol 1-*tert*-Butoxypropanol-2, 0,06 mmol (Ph₅Cp)Ru(CO)₂Cl, Novozym 435 33 mg, 0,1 mmol t-BuOK vermischt. Um den Einfluss von Na₂CO₃ auf den Umsatz der Reaktion zu ermitteln, wurde die Konzentration an Na₂CO₃ variiert. Die Ergebnisse sind in Tabelle 4 dargestellt:

**Tabelle 4:**

| Lauf | Zeit [h] | Na₂CO₃ [mg] | Umsatz [%] | e.e. [%] |
|---|---|---|---|---|
| 1 | 48 | 50 | 65 | 99 |
| 2 | 120 | 50 | 85 | 99 |
| 3 | 48 | 150 | 85 | 99 |
| 4 | 120 | 150 | 92 | 99 |

| | | | | |
|---|---|---|---|---|
| Aus Tabelle 4 geht hervor, dass in Gegenwart größerer Mengen der Base Na₂CO₃ die Reaktion deutlich schneller abläuft. So kann nach 48 Stunden in Gegenwart von 50 mg ein Umsatz von 65 % erzielt werden (Lauf 1), während mit 150 mg Na₂CO₃ und der gleichen Menge Katalysator und Enzym ein Umsatz von 85 % erzielt werden kann (Lauf 4). | | | | |

### Beispiel 7: Dynamisch kinetische Racematspaltung von 1-tert-Butoxypropanol-2 im Grammmaßstab

In einem 50 mL Schlenkgefäß mit einem Magnetrührfisch wurden Chlorodicarbonyl(1,2,3,4,5-pentaphenylcyclopentadienyl)ruthenium (40 mg, 0.06 mmol), immobilisierte CALB von Aldrich (33 mg) und Na₂CO₃ (0,15 g, 1.4 mmol) gegeben. Das Gefäß wurde evakuiert und mit Argon gefüllt. Toluol (20 mL) wurde in einer Argonatmosphäre zugegeben. Die Reaktionsmischung wurde bei Raumtemperatur gerührt, bis der Rutheniumkomplex gelöst war. Danach wurde eine Lösung von *^{t}*BuOK in THF (1 M) (0.1 mL, 0.1 mmol) zugegeben und die Reaktionsmischung weitere 6 Minuten gerührt. 1-*tert*-Butoxypropanol-2 (2.62 g, 3 ml, 19.8 mol) wurde zur entstandenen Mischung zugegeben und die Reaktionsmischung über weitere 4 Minuten gerührt. Anschließend wurde Isopropenylacetat (2.00 g, 20 mol) bei Raumtemperatur zugegeben und die Reaktionsmischung auf 75 °C erhitzt. Nach 120 h wurde eine Probe genommen und mit Hilfe der GC (HP-5, 50 m) analysiert. Laut dieser Analyse wurden 93 % Umsatz erreicht. Die Reaktionsmischung wurde zum Schluss abgekühlt, durch einen Papierfilter filtriert und unter einem verminderten Druck von 20 mbar aufkonzentriert. Der Rückstand wurde unter Vaccum destilliert (80 °C, 5 mbar). (R)-2-O-Acetyl-1-O-*tert*-butyl-propan-1,2-diol 2.15 g (63 % Ausbeute, 99.5%ee) wurde als farblose Flüssigkeit erhalten.

Vorteile, die mit dem erfindungsgemäßen Verfahren einhergehen:
- Herstellung von Propan-1,2-diol in einer optischen Reinheit von > 99 % e.e. ausgehend von Lactiden, dabei ist es auch möglich von meso-Lactid auszugehen, das als Abfallprodukt bei der Milchsäurepolymerisation anfällt

## Patentansprüche

1. Verfahren zur Herstellung von optisch reinem Propan-1,2-diol umfassend die Verfahrensschritte
a. Hydrierung von Lactiden, wobei eine metallkatalysierte heterogene Katalyse in Gegenwart von Wasserstoff durchgeführt wird, wobei ein Rohprodukt, enthaltend Propan-1,2-diol, hergestellt wird, und
b. dynamisch kinetische Racematspaltung, wobei in einem Bereich von ≥ 99 % e.e. optisch reines Propan-1,2-diol hergestellt wird.

2. Verfahren nach Anspruch 1, **dadurch gekennzeichnet, dass** die Lactide ausgewählt werden aus der Gruppe umfassend D,D-Lactid, L, L-Lactid, meso-Lactid und L,L/D,D-Lactid.

3. Verfahren nach einem der Ansprüche 1 oder 2, **dadurch gekennzeichnet, dass** die metallkatalysierte heterogene Katalyse in Schritt a) in der Flüssigphase durchgeführt wird.

4. Verfahren nach Anspruch 3, **dadurch gekennzeichnet, dass** die Flüssigphase ausgewählt wird aus einer Gruppe Lösungsmittel umfassend, Wasser, aliphatische oder aromatische Kohlenwasserstoffe aufweisend eine Kettenlänge von bis zu 10 C-Atomen und Mischungen davon, wobei die aliphatischen Kohlenwasserstoffe bevorzugt Alkohole sind, und besonders bevorzugt Methanol und/oder Ethanol eingesetzt werden.

5. Verfahren nach einem der Ansprüche 1 bis 4, **dadurch gekennzeichnet, dass** die heterogene Katalyse in Schritt a) mittels eines Katalysators aus der Gruppe der Metalle durchgeführt wird, wobei das Metall aus einer Gruppe umfassend Ruthenium, Rhodium, Rhenium, Palladium, Platin, Nickel, Kobalt, Molybdän, Wolfram, Titan, Zirkonium, Niobium, Vanadium, Chrom, Mangan, Osmium, Iridium, Eisen, Kupfer Zink, Silber, Gold, Barium und Mischungen davon ausgewählt wird, und bevorzugt Kupferchromit-Katalysatoren und/oder mit Barium versetzte Kupferchromit-Katalysatoren eingesetzt werden.

6. Verfahren nach einem der vorstehenden Ansprüche, **dadurch gekennzeichnet, dass** die heterogene Katalyse in Schritt a) unter 20 bis 300 bar Wasserstoffdruck, bevorzugt unter 130 bis 170 bar Wasserstoffdruck und besonders bevorzugt unter 140 bis 160 bar Wasserstoffdruck durchgeführt wird.

7. Verfahren nach einem der vorstehenden Ansprüche, **dadurch gekennzeichnet, dass** die heterogene Katalyse in Schritt a) in einem Temperaturbereich von 20°C bis 250°C, bevorzugt in einem Temperaturbereich von 130°C bis 170°C, und besonders bevorzugt in einem Temperaturbereich von 145°C bis 155°C durchgeführt wird.

8. Verfahren nach einem der vorstehenden Ansprüche, **dadurch gekennzeichnet, dass** vor der Durchführung der heterogenen Katalyse in Schritt a) der Druckbehälter 1 bis 5 mal und bevorzugt 3 mal mit Wasserstoff gespült wird.

9. Verfahren nach einem der vorstehenden Ansprüche, **dadurch gekennzeichnet, dass** die heterogene Katalyse in Schritt a) über einen Zeitraum von 5 bis 20 Stunden, bevorzugt über einen Zeitraum von 10 bis 18 Stunden, und besonders bevorzugt über einen Zeitraum von 12 bis 16 Stunden durchgeführt wird.

10. Verfahren nach einem der vorstehenden Ansprüche, **dadurch gekennzeichnet, dass** bei der heterogenen Katalyse in Schritt a) gerührt wird.

11. Verfahren nach einem der vorstehenden Ansprüche, **dadurch gekennzeichnet, dass** bei der heterogenen Katalyse in Schritt a) kontinuierlich Wasserstoff nachgepresst wird.

12. Verfahren nach einem der vorstehenden Ansprüche, **dadurch gekennzeichnet, dass** der Katalysator nach Beenden der heterogenen Katalyse in Schritt a) vom Rohprodukt getrennt wird.

13. Verfahren nach einem der vorstehenden Ansprüche, **dadurch gekennzeichnet, dass** das Rohprodukt, das aus Schritt a) resultiert, einem Aufkonzentrierungsschritt und/oder einem Destillationsschritt unterzogen wird, wobei eine Fraktion enthaltend Propan-1,2-diol und eine Fraktion enthaltend Lösungsmittel generiert werden.

14. Verfahren nach einem der vorstehenden Ansprüche, **dadurch gekennzeichnet, dass** das Lösungsmittel, das zur heterogenen Katalyse in Schritt a) eingesetzt wird, in das Verfahren zurückgeführt wird.

15. Verfahren nach einem der vorstehenden Ansprüche, **dadurch gekennzeichnet, dass** das Propan-1,2-diol, das aus Schritt a) erhalten wird, mit einer Schutzgruppe versehen wird und 1-*O*-substituiertes Propandiol erzeugt wird.

16. Verfahren nach Anspruch 15, **dadurch gekennzeichnet, dass** die Schutzgruppe eine rezyklisierbare, achirale Schutzgruppe ist und ausgewählt wird aus der Gruppe umfassend *tert*-Butyl, Phenyl, Methyl, Acetyl, Benzoyl, Trityl, Silyl und Benzyl.

17. Verfahren nach einem der vorstehenden Ansprüche, **dadurch gekennzeichnet, dass** in Schritt b) zur dynamisch kinetischen Racematspaltung eine enzymatische Racematspaltung in Gegenwart eines Metallkatalysators durchgeführt wird.

18. Verfahren nach Anspruch 17, **dadurch gekennzeichnet, dass** bei der enzymatischen Racematspaltung Lipasen eingesetzt werden.

19. Verfahren nach einem der Ansprüche 17 oder 18, **dadurch gekennzeichnet, dass** Rutheniumkatalysatoren als Metallkatalysatoren eingesetzt werden.

20. Verfahren nach einem der Ansprüche 17 bis 19, **dadurch gekennzeichnet, dass** die dynamische kinetische Racematspaltung mittels Rutheniumkatalysatoren mit immobilisierten Lipasen durchgeführt wird.

21. Verfahren nach einem der vorstehenden Ansprüche, **dadurch gekennzeichnet, dass** die dynamisch kinetische Racematspaltung in Schritt b) in einem Temperaturbereich von 60°C bis 90°C durchgeführt wird.

22. Verfahren nach einem der vorstehenden Ansprüche, **dadurch gekennzeichnet, dass** die dynamisch kinetische Racematspaltung in Schritt b) über einen Zeitraum von 30 bis 200 h, bevorzugt in einem Zeitraum von in 40 bis 60 h durchgeführt wird.

23. Verfahren nach einem der vorstehenden Ansprüche, **dadurch gekennzeichnet, dass** die dynamisch kinetische Racematspaltung in Schritt b) in Gegenwart von Na₂CO₃ durchgeführt wird, wobei das Na₂CO₃ in Mengen von 0,4 mmol bis 5 mmol pro 33 mg Enzym zugesetzt wird.

## Claims

1. Process for the production of optically pure propane-1,2-diol comprising the following process steps:
a. Hydrogenation of lactides, wherein a metal-catalysed heterogeneous catalysis is carried out in the presence of hydrogen, a raw product containing propane-1,2-diol being produced, and
b. Dynamic kinetic racemic resolution, in which optically pure propane-1.2-diol is produced within a range of ≥ 99% e.e..

2. Process in accordance with Claim 1, **characterised in that** the lactides are selected from the group comprising D,D-lactide, L, L-lactide, meso-lactide and L,L/D,D-lactide.

3. Process in accordance with one of Claims 1 and 2, **characterised in that** the metal-catalysed heterogeneous catalysis in step a) is carried out in the liquid phase.

4. Process in accordance with Claim 3, **characterised in that** the liquid phase is selected from a group of solvents comprising water, aliphatic or aromatic hydrocarbons with a chain length of up to 10 C-atoms, and mixtures thereof, wherein the aliphatic hydrocarbons are preferably alcohols with particular preference being given to methanol and/or ethanol being used.

5. Process in accordance with one of Claims 1 to 4, **characterised in that** the heterogeneous catalysis in step a) is carried out using a catalyst from the metals group, wherein the metal is selected from a group comprising ruthenium, rhodium, rhenium, palladium, platinum, nickel, cobalt, molybdenum, wolfram, titanium, zirconium, niobium, vanadium, chromium, manganese, osmium, iridium, iron, copper, zinc, silver, gold, barium and mixtures thereof, preference being given to the use of copper-chromite catalysts and/or copper-chromite catalysts with barium added.

6. Process in accordance with one of the above claims, **characterised in that** the heterogeneous catalysis in step a) is carried out at a hydrogen pressure of 20 to 300 bar, with preference given to a hydrogen pressure of 130 to 170 bar, and particular preference given to a hydrogen pressure of 140 to 160 bar.

7. Process in accordance with one of the above claims, **characterised in that** the heterogeneous catalysis in step a) is carried out within a temperature range of 20°C to 250°C, preferably within a temperature range of 130°C to 170°C, with particular preference given to a temperature range of 145°C to 155°C.

8. Process in accordance with one of the above claims, **characterised in that** prior to the heterogeneous catalysis in step a) being carried out, the pressure vessel is rinsed 1 to 5 times, preferably 3 times, with hydrogen.

9. Process in accordance with one of the above claims, **characterised in that** the heterogeneous catalysis is carried out in step a) over a period of 5 to 20 hours, preferably over a period of 10 to 18 hours, with particular preference given to a period of 12 to 16 hours.

10. Process in accordance with one of the above claims, **characterised in that** agitation occurs during the heterogeneous catalysis in step a).

11. Process in accordance with one of the above claims, **characterised in that** hydrogen is continuously replenished during the heterogeneous catalysis in step a).

12. Process in accordance with one of the above claims, **characterised in that** the catalyst is separated off from the raw product once the heterogeneous catalysis in step a) has been completed.

13. Process in accordance with one of the above claims, **characterised in that** the raw product resulting from step a) is subjected to a concentration step and/or a distillation step, wherein a fraction containing propane-1,2-diol and a fraction containing solvent are generated.

14. Process in accordance with one of the above claims, **characterised in that** the solvent used for the heterogeneous catalysis in step a) is fed back into the process.

15. Process in accordance with one of the above claims, **characterised in that** the propane-1,2-diol which is obtained from step a) is furnished with a protective group and 1-*O*-substituted propanediol is produced.

16. Process in accordance with Claim 15, **characterised in that** the protective group is a recyclable, achiral protective group and is selected from the group comprising *tert*-butyl, phenyl, methyl, acetyl, benzoyl, trityl, silyl and benzyl.

17. Process in accordance with one of the above claims, **characterised in that** an enzymatic racemic resolution is carried out for the dynamic kinetic racemic resolution in the presence of a metal catalyst during step b).

18. Process in accordance with Claim 17, **characterised in that** lipases are used during the enzymatic racemic resolution.

19. Process in accordance with one of Claims 17 and 18, **characterised in that** ruthenium catalysts are used as metal catalysts.

20. Process in accordance with one of Claims 17 to 19, **characterised in that** the dynamic kinetic racemic resolution is carried out using ruthenium catalysts with immobilised lipases.

21. Process in accordance with one of the above claims, **characterised in that** the dynamic kinetic racemic resolution is carried out in step b) within a temperature range of 60°C to 90°C.

22. Process in accordance with one of the above claims, **characterised in that** the dynamic kinetic racemic resolution in step b) is carried out over a period of 30 to 200 hrs, preferably within a period of 40 to 60 hrs.

23. Process in accordance with one of the above claims, **characterised in that** the dynamic kinetic racemic resolution in step b) is carried out in the presence of Na₂CO₃, wherein the Na₂CO₃ is added in quantities of 0.4 mmol to 5 mmol per 33 mg of enzyme.

## Revendications

1. Procédé de fabrication de propane-1,2-diol optiquement pur, comprenant les étapes de procédé suivantes :
a. l'hydrogénation de lactides, une catalyse hétérogène catalysée par un métal étant réalisée en présence d'hydrogène, un produit brut contenant du propane-1,2-diol étant fabriqué, et
b. le clivage cinétique dynamique du racémat, du propane-1,2-diol optiquement pur dans une plage de ≥ 99 % d'e.e. étant fabriqué.

2. Procédé selon la revendication 1, **caractérisé en ce que** les lactides sont choisis dans le groupe comprenant le D,D-lactide, le L,L-lactide, le méso-lactide et le L,L/D,D-lactide.

3. Procédé selon l'une quelconque des revendications 1 ou 2, **caractérisé en ce que** la catalyse hétérogène catalysée par un métal à l'étape a) est réalisée en phase liquide.

4. Procédé selon la revendication 3, **caractérisé en ce que** la phase liquide est choisie dans le groupe de solvants comprenant l'eau, les hydrocarbures aliphatiques ou aromatiques présentant une longueur de chaîne de jusqu'à 10 atomes C et leurs mélanges, les hydrocarbures aliphatiques étant de préférence des alcools, et le méthanol et/ou l'éthanol étant utilisés de manière particulièrement préférée.

5. Procédé selon l'une quelconque des revendications 1 à 4, **caractérisé en ce que** la catalyse hétérogène à l'étape a) est réalisée au moyen d'un catalyseur du groupe des métaux, le métal étant choisi dans un groupe comprenant le ruthénium, le rhodium, le rhénium, le palladium, le platine, le nickel, le cobalt, le molybdène, le tungstène, le titane, le zirconium, le niobium, le vanadium, le chrome, le manganèse, l'osmium, l'iridium, le fer, le cuivre, le zinc, l'argent, l'or, le baryum et leurs mélanges, et des catalyseurs de chromite de cuivre et/ou des catalyseurs de chromite de cuivre mélangés avec du baryum étant utilisés de préférence.

6. Procédé selon l'une quelconque des revendications précédentes, **caractérisé en ce que** la catalyse hétérogène à l'étape a) est réalisée sous 20 à 300 bar de pression d'hydrogène, de préférence sous 130 à 170 bar de pression d'hydrogène et de manière particulièrement préférée sous 140 à 160 bar de pression d'hydrogène.

7. Procédé selon l'une quelconque des revendications précédentes, **caractérisé en ce que** la catalyse hétérogène à l'étape a) est réalisée dans une plage de température allant de 20 °C à 250 °C, de préférence dans une plage de température allant de 130 °C à 170 °C, et de manière particulièrement préférée dans une plage de température allant de 145 °C à 155 °C.

8. Procédé selon l'une quelconque des revendications précédentes, **caractérisé en ce que** le contenant sous pression est rincé 1 à 5 fois et de préférence 3 fois avec de l'hydrogène avant la réalisation de la catalyse hétérogène à l'étape a).

9. Procédé selon l'une quelconque des revendications précédentes, **caractérisé en ce que** la catalyse hétérogène à l'étape a) est réalisée pendant une durée de 5 à 20 heures, de préférence pendant une durée de 10 à 18 heures, et de manière particulièrement préférée pendant une durée de 12 à 16 heures.

10. Procédé selon l'une quelconque des revendications précédentes, **caractérisé en ce qu'**une agitation est réalisée pendant la catalyse hétérogène à l'étape a).

11. Procédé selon l'une quelconque des revendications précédentes, **caractérisé en ce que** de l'hydrogène est injecté en continu pendant la catalyse hétérogène à l'étape a).

12. Procédé selon l'une quelconque des revendications précédentes, **caractérisé en ce que** le catalyseur est séparé du produit brut après la fin de la catalyse hétérogène à l'étape a).

13. Procédé selon l'une quelconque des revendications précédentes, **caractérisé en ce que** le produit brut qui résulte de l'étape a) est soumis à une étape de concentration et/ou à une étape de distillation, une fraction contenant du propane-1,2-diol et une fraction contenant le solvant étant générées.

14. Procédé selon l'une quelconque des revendications précédentes, **caractérisé en ce que** le solvant qui est utilisé pour la catalyse hétérogène à l'étape a) est recyclé dans le procédé.

15. Procédé selon l'une quelconque des revendications précédentes, **caractérisé en ce que** le propane-1,2-diol qui est obtenu à l'étape a) est muni d'un groupe protecteur et du propanediol 1-0-substitué est généré.

16. Procédé selon la revendication 15, **caractérisé en ce que** le groupe protecteur est un groupe protecteur achiral recyclisable et est choisi dans le groupe comprenant tert-butyle, phényle, méthyle, acétyle, benzoyle, trityle, silyle et benzyle.

17. Procédé selon l'une quelconque des revendications précédentes, **caractérisé en ce qu'**un clivage enzymatique du racémat en présence d'un catalyseur métallique est réalisé à l'étape b) pour le clivage cinétique dynamique du racémat.

18. Procédé selon la revendication 17, **caractérisé en ce que** des lipases sont utilisées lors du clivage enzymatique du racémat.

19. Procédé selon l'une quelconque des revendications 17 ou 18, **caractérisé en ce que** des catalyseurs de ruthénium sont utilisés en tant que catalyseurs métalliques.

20. Procédé selon l'une quelconque des revendications 17 à 19, **caractérisé en ce que** le clivage cinétique dynamique du racémat est réalisée au moyen de catalyseurs de ruthénium avec des lipases immobilisées.

21. Procédé selon l'une quelconque des revendications précédentes, **caractérisé en ce que** le clivage cinétique dynamique du racémat à l'étape b) est réalisé dans une plage de température allant de 60 °C à 90 °C.

22. Procédé selon l'une quelconque des revendications précédentes, **caractérisé en ce que** le clivage cinétique dynamique du racémat à l'étape b) est réalisé pendant une durée de 30 à 200 h, de préférence pendant une durée de 40 à 60 h.

23. Procédé selon l'une quelconque des revendications précédentes, **caractérisé en ce que** le clivage cinétique dynamique du racémat à l'étape b) est réalisé en présence de Na₂CO₃, le Na₂CO₃ étant ajouté en quantités de 0,4 mmol à 5 mmol pour 33 mg d'enzyme.
